# EUROPEAN PATENT APPLICATION

(11) **EP 4 810 071 A1**
(43) Date of publication of application: **23.09.2026**
(21) Application number: 25213109.9
(22) Date of filing: 03.11.2025
(51) Int. Cl.: A61L 9/12

(54) **FRAGRANCE DIFFUSING ASSEMBLY AND FRAGRANCE DIFFUSER**

(30) Priority: 17.03.2025 CN 202520467944 U
(71) Applicant: Beijing Xiaomi Mobile Software Co., Ltd., Beijing 100085 (CN)
(72) Inventor: WEI, Jinggang, Beijing (CN); WANG, Chong, Beijing (CN); LIU, Zunfeng, Beijing (CN)
(74) Representative: Dehns Germany Partnerschaft mbB

(57) **Abstract**

A fragrance diffusing assembly includes an oil storage member (2) having an oil storage cavity (21) for storing fragrance liquid; a mounting bracket (3) assembled at an open end of the oil storage cavity and including an annular wall (35), and the annular wall encloses a fragrance spreading cavity (31) in communication with the oil storage cavity (21); a sealing top cover (5) rotatably arranged at a top of the annular wall and is in elastic contact seal with a top end face and an outer wall surface of the annular wall; when the sealing top cover is in an open position, the fragrance in the fragrance spreading cavity diffuses to the outside of the fragrance diffusing assembly, and when the sealing top cover is in the closed position, the sealing top cover seals the fragrance spreading cavity.

## Description

### FIELD

The present invention relates to relates to the technical field of terminal technologies, in particular to a fragrance diffusing assembly and a fragrance diffuser.

### BACKGROUND

Nowadays, an on-board fragrance diffuser has become a popular accessory for automobiles.

### SUMMARY

The present invention provides a fragrance diffusing assembly and a fragrance diffuser to solve the deficiencies in the related art.

According to a first aspect of the present invention, there is provided a fragrance diffusing assembly, including:
an oil storage member having an oil storage cavity for storing fragrance liquid;
a mounting bracket assembled at an open end of the oil storage cavity, and including an annular wall enclosing a fragrance spreading cavity in communication with the oil storage cavity; and
a sealing top cover rotatably arranged at a top of the annular wall and in elastic contact seal with a top end face and an outer wall surface of the annular wall; and when the sealing top cover is in an open position, fragrance in the fragrance spreading cavity diffuses to outside of the fragrance diffusing assembly; and when the sealing top cover is in a closed position, the sealing top cover seals the fragrance spreading cavity.

Optionally, a contact surface between the sealing top cover and the top end face of the annular wall and a contact surface between the sealing top cover and the outer wall surface of the annular wall are elastic contact surfaces.

Optionally, the sealing top cover includes a hard outer cover and a flexible sealing sleeve fixedly arranged in the hard outer cover, and the flexible sealing sleeve is in elastic sealing contact with the top end face and the outer wall surface of the annular wall.

Optionally, the hard outer cover includes a first skirt;
the flexible sealing sleeve includes a second skirt fitted with an inner side of the first skirt, and the second skirt is in elastic sealing contact with the outer wall surface of the annular wall.

Optionally, the first skirt has a first through hole, the second skirt has a notch and a second through hole, and the first through hole is in communication with the second through hole;
the notch forms a ventilation groove in communication with the fragrance spreading cavity, and the first through hole and the second through hole form a diffusion opening in communication with the fragrance spreading cavity.

Optionally, the sealing top cover further includes a rotating outer frame rotatably installed on the mounting bracket and located at an outer periphery of the hard outer cover, and the rotating outer frame is configured to drive the hard outer cover and the flexible sealing sleeve to rotate under action of an external force.

Optionally, the mounting bracket further includes a ventilation channel arranged outside the fragrance spreading cavity, the annular wall is located between the ventilation channel and the fragrance spreading cavity, and the annular wall further includes an air inlet and a fragrance spreading opening;
the sealing top cover includes a ventilation groove and a diffusion opening, wherein the ventilation groove is in communication with the ventilation channel and the diffusion opening is in communication with the outside of the fragrance diffusing assembly; when the sealing top cover is turned to the open position, the ventilation groove is in communication with the air inlet and the fragrance spreading opening is in communication with the diffusion opening; when the sealing top cover is turned to the closed position, the ventilation groove is completely staggered from the air inlet, and the fragrance spreading opening is completely staggered from the diffusion opening, so that the sealing top cover realizes elastic sealing of the fragrance spreading opening and the air inlet of the annular wall.

Optionally, the ventilation channel is in communication with both the fragrance spreading opening and the air inlet, and the fragrance spreading opening is configured to coincide with the air inlet after rotating 180 degrees around an axial direction of the annular wall.

Optionally, the annular wall includes an air inlet in communication with the fragrance spreading cavity; and the fragrance diffusing assembly further includes:
a housing, wherein the oil storage member is installed in the housing and located at a side of the housing, an air inlet channel is formed at another side of the housing and in communication with the air inlet and the outside of the fragrance diffusing assembly, and the air inlet channel is formed by an outer wall of the oil storage member and an inner wall of the housing.

Optionally, the fragrance diffusing assembly further includes a hard fiber fragrance spreading stick and a sealing ring, wherein the sealing ring seals the open end of the oil storage cavity and is connected to the mounting bracket; the hard fiber fragrance spreading stick extends out from the oil storage member through the sealing ring and is partially located in the fragrance spreading cavity; and the hard fiber fragrance spreading stick is in interference fit with the sealing ring.

According to a second aspect of the present invention, there is provided a fragrance diffuser, including: the fragrance diffusing assembly according to any of the above embodiments; and a main unit electrically connected with the fragrance diffusing assembly.

The technical solution provided by embodiments of the present invention includes the following beneficial effects.

It may be seen from the above embodiments that through the technical solution of the present invention, the fragrance spreading cavity is sealed by the elastic contact seal between the sealing top cover and the annular wall, so that the sealing performance of the fragrance spreading cavity is improved, and the risks of liquid leakage and leakage during transportation are reduced.

It is to be understood that both the foregoing general description and the following detailed description are illustrative and explanatory only and are not restrictive of the present invention.

### BRIEF DESCRIPTION OF THE DRAWINGS

The accompanying drawings, which are incorporated in and constitute a part of the specification, illustrate embodiments consistent with the present invention and together with the description, serve to explain the principles of the invention.
FIG. 1 is an exploded schematic view of a fragrance diffusing assembly according to an illustrative embodiment.
FIG. 2 is a schematic cross-sectional view of the fragrance diffusing assembly in FIG. 1.
FIG. 3 is a schematic structural view of a sealing top cover according to an illustrative embodiment.
FIG. 4 is another schematic cross-sectional view of the fragrance diffusing assembly in FIG. 1.
FIG. 5 is a partial schematic view of the fragrance diffusing assembly in FIG. 4.
FIG. 6 is another schematic sectional view of the fragrance diffusing assembly in FIG. 1.
FIG. 7 is a partial schematic view of the fragrance diffusing assembly in FIG. 6.

### DETAILED DESCRIPTION

Reference will now be made in detail to illustrative embodiments, examples of which are illustrated in the accompanying drawings. When the following description refers to the drawings, unless otherwise indicated, the same numbers in different drawings indicate the same or similar elements. The embodiments described in the following illustrative embodiments do not represent all embodiments consistent with the present invention. Rather, they are merely examples of devices and methods consistent with some aspects of the present invention as detailed in the appended claims.

The terminology used in the present invention is for the purpose of describing specific embodiments only and is not intended to limit the present invention. The singular forms "a", "said" and "the" used in the present invention and the appended claims are also intended to include plural forms, unless the context clearly indicates other meaning. It should also be understood that the term "and/or" as used herein refers to and includes any or all possible combinations of one or more associated listed items.

It should be understood that although the terms "first", "second", "third", etc. may be used to describe various information in the present invention, these information should not be limited to these terms. These terms are only used to distinguish the same type of information from each other. For example, without departing from the scope of the present invention, the first information may also be called the second information, and similarly, the second information may also be called the first information. Depending on the context, the word "if" as used herein may be interpreted as "at the time of" or "when" or "in response to".

In related arts, sealing to a fragrance diffuser cavity of fragrance diffusing assembly is mainly rigid contact sealing, which leads to poor sealing performance, which easily leads to liquid leakage and leakage during transportation of fragrance diffusing assembly's accessories.

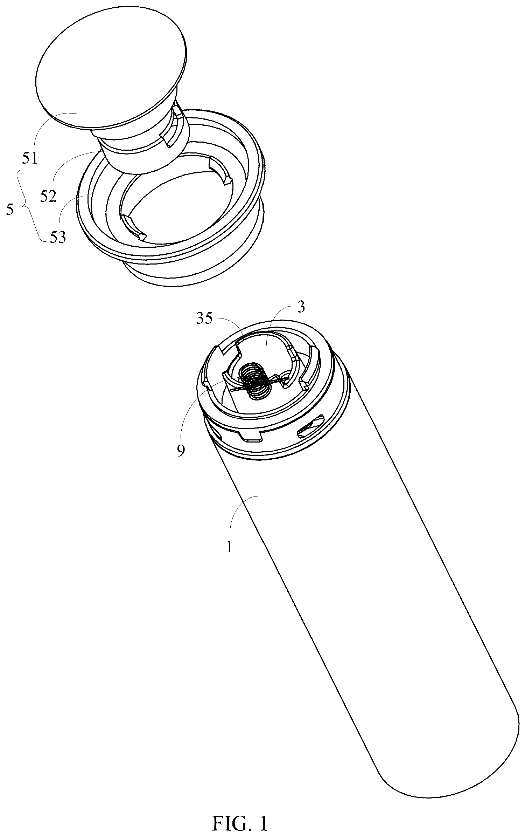

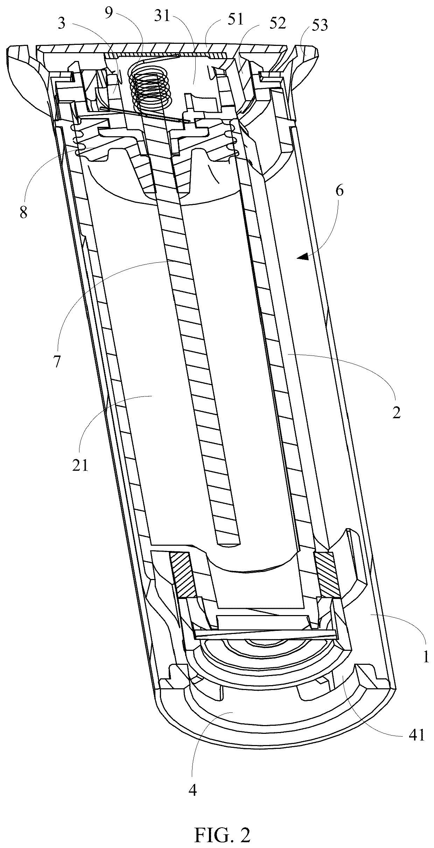

FIG. 1 is an exploded schematic view of a fragrance diffusing assembly according to an illustrative embodiment, and FIG. 2 is a sectional schematic view of the fragrance diffusing assembly in FIG. 1. As shown in FIGS. 1 and 2, the fragrance diffusing assembly includes a housing 1, an oil storage member 2, a mounting bracket 3, a base 4 and a sealing top cover 5. The oil storage member 2 is fixedly arranged in the housing 1, and the base 4 is fixedly arranged below the housing 1 and the oil storage member 2, and the base 4 may be used as a support for the fragrance diffusing assembly. The oil storage member 2 is installed in the housing 1, and a gap between the outer wall of the oil storage member 2 and the inner wall of the housing 1 forms an air inlet channel 6 in communication with the outside of the fragrance diffusing assembly. For example, the air inlet channel 6 may surround an outer periphery of the oil storage member 2, that is, the air inlet channel 6 may be an annular air channel. For another example, the oil storage member 2 is arranged on a side of the housing 1, and the air inlet channel 6 is formed on another side of the housing 1, so that the oil storage member 2 may be arranged as close as possible to one side of the housing 1, so that the oil storage volume of the oil storage member 2 may be increased relative to the structure of the annular air channel, thereby increasing the oil storage capacity. The base 4 and the oil storage member 2 are relatively fixed, and the base 4 has an air inlet 41 in communication with the air inlet channel 6, that is, the external air flow may enter the air inlet channel 6 through the air inlet 41.

The oil storage member 2 has an oil storage cavity 21 for storing fragrance liquid, and the mounting bracket 3 is relatively fixed with the oil storage member 2. The mounting bracket 3 may be relatively fixed with the housing 1. The mounting bracket 3 is assembled at the open end of the oil storage cavity 21, and the mounting bracket 3 and the oil storage member 2 may be relatively fixed by clamping or interference fit. The mounting bracket 3 includes an annular wall 35, which may enclose a fragrance spreading cavity 31 in communication with the oil storage cavity 21, and the fragrance in the oil storage cavity 21 may spread to the fragrance spreading cavity 31 and then further spread to the outside of the fragrance diffusing assembly. The annular wall 35 is mainly defined as a vertical wall surrounding a depth direction of the fragrance spreading cavity 31, and the shape of the annular wall 35 is not specifically limited here. In fact, the annular wall 35 may be adaptively designed according to the shape requirements of the fragrance spreading cavity 31.

The sealing top cover 5 may be rotatably installed at the top of the annular wall 35, and the sealing top cover 5 is in elastic contact seal with the top end face and the outer wall surface of the annular wall 35. When the sealing top cover 5 is in the open position, the fragrance in the fragrance spreading cavity 31 may be diffused to the outside of the fragrance diffusing assembly, and when the sealing top cover 5 is in the closed position, the fragrance-spreading cavity 31 may be sealed through the sealing top cover 5. Thus, the elastic contact seal between the sealing top cover 5 and the annular wall 35 may be used to improve the sealing performance of the fragrance-spreading cavity 31 and reduce the risk of liquid leakage and leakage during transportation.

In some embodiments, the top end face and the outer wall surface of the annular wall 35 are both elastic contact surfaces, so as to realize elastic contact sealing between the sealing top cover 5 and the annular wall 35.

In other embodiments, the contact surface of the sealing top cover 5 in contact with the top end face of the annular wall 35 and the contact surface of the sealing top cover 5 in contact with the outer wall surface of the annular wall 35 are all elastic contact surfaces, so as to realize elastic contact sealing between the sealing top cover 5 and the annular wall 35. The sealing top cover 5 may be an elastic member, so that the contact surfaces of the sealing top cover 5 in contact with the top end face and the outer wall surface of the annular wall 35 are elastic contact surfaces, or the sealing top cover 5 may be partially made of an elastic member, and the elastic member of the sealing top cover 5 contacts with the top end face and the outer wall surface of the annular wall 35.

In the above embodiments, the sealing top cover 5 may include a hard outer cover 51 and a flexible sealing sleeve 52 fixed in the hard outer cover 51, and the flexible sealing sleeve 52 is in elastic sealing contact with the top end face and the outer wall surface of the annular wall 35. The flexible sealing sleeve 52 may include a rubber sleeve, such as a plastic sleeve or a rubber sleeve, etc. The hard outer cover 51 and the flexible sealing sleeve 52 may be bonded or integrally fixed with each other, and their fixing method is not limited. On the one hand, the design of sealing top cover 5 combining the hard outer cover 51 with the flexible sealing sleeve 52 may ensure elastic contact sealing, on the other hand, the design improves the strength of the sealing top cover 5, which is beneficial to the smoothness of switching of the sealing top cover 5 between the open position and the closed position.

In order to switch between the open position and the closed position, the sealing top cover 5 further includes a rotating outer frame 53 rotatably installed on the mounting bracket 3 and arranged on the outer periphery of the hard outer cover 51, and the rotating outer frame 53 is configured to drive the hard outer cover 51 and the flexible sealing sleeve 52 to rotate under the action of external force. The rotating outer frame 53 and the hard outer cover 51 may be clamped and connected with each other, and the hard outer cover 51 and the flexible sealing sleeve 52 may be driven to rotate relative to the mounting bracket 3 by rotating the outer frame 53, and the relative position between the flexible sealing sleeve 52 and the annular wall 35 may be switched, so as to realize the opening and closing of the fragrance spreading cavity 31.

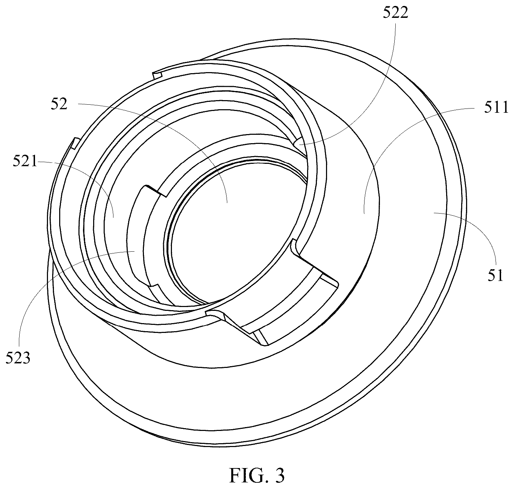

In some embodiments, as shown in FIG. 3, the hard outer cover 51 includes a first skirt 511, and the flexible sealing sleeve 52 includes a second skirt 521 fitted with the inner side of the first skirt 511 and in elastic sealing contact with the outer wall surface of the annular wall 35. For example, the first skirt 511 may be an annular skirt, and the second skirt 521 may also be an annular skirt matched with the outer wall surface of the annular wall 35. By rotating the second skirt 521 along the outer wall surface of the annular wall 35, the fragrance spreading cavity 31 may be opened or closed. For another example, the first skirt 511 and the second skirt 521 may also be arc-shaped skirts, and the area of the arc-shaped skirts may be designed to seal the fragrance spreading cavity 31 in the closed position. Specifically, the area of the arc-shaped skirts may be designed as required, which are not limited in the present invention.

In order to open and close the fragrance spreading cavity 31, the first skirt 511 has a first through hole 512, and the second skirt 521 has a notch 522 and a second through hole 523. The first through hole 512 and the second through hole 523 is in communication with each other. For example, the first through hole 512 and the second through hole 523 may be through holes of the same size and aligned with each other. For another example, the first through hole 512 and the second through hole 523 may be different in size and partially aligned to realize air flow. When the sealing top cover 5 is in the open position, the notch 522 may be a ventilation groove in communication with the fragrance spreading cavity 31, and the first through hole 512 and the second through hole 523 form a diffusion opening in communication with the fragrance spreading cavity 31 and the outside of the fragrance diffusing assembly. The notch 522 may also be in communication with the air inlet channel 6, so that natural wind may enter the fragrance spreading cavity 31 through the ventilation groove, and diffuse through the diffusion opening after mixing with the fragrance flavor, so as to enter the outside of the fragrance diffusing assembly and realize fragrance spreading. The structural differences between the ventilation groove and the diffusion opening make the natural wind entering the air inlet channel 6 always enter through the ventilation groove, and diffuses through the diffusion opening so as to realize the consistency of the design of the air path and prevent the airflow from straggling inside of the fragrance diffusing assembly and affecting the fragrance spreading effect.

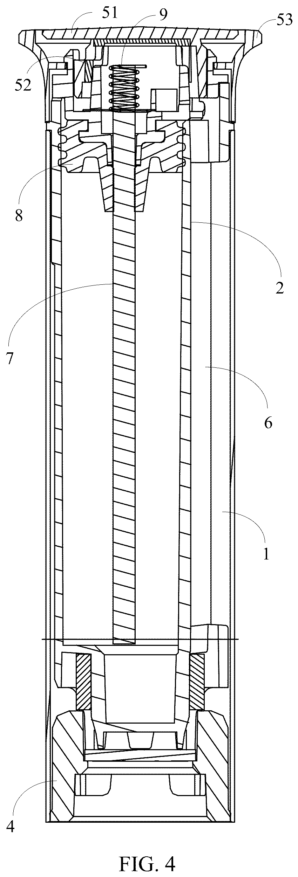

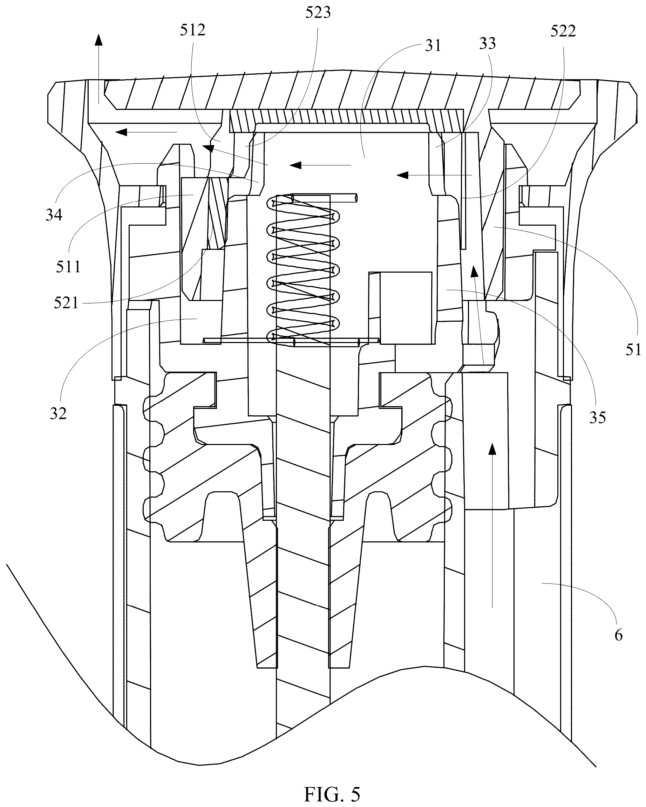

As shown in FIG. 4 and FIG. 5, aiming at the air channel design of fragrance diffusing assembly in the present invention, the mounting bracket 3 further includes an air channel 32 arranged at an outer side of the fragrance spreading cavity 31, and the annular wall 35 is arranged between the fragrance spreading cavity 31 and the air channel 32, that is, it may be understood that the inner wall of the annular wall 35 may form the inner wall of the fragrance spreading cavity 31, and the outer wall surface of the annular wall 35 may form the inner wall of the air channel 32. The notch 522 on the second skirt 521 of the flexible sealing sleeve 52 acts as a ventilation groove to be in communication with the ventilation channel 32. The annular wall 35 also includes the air inlet 33 and the fragrance spreading opening 34. When the sealed top cover 5 is in the open position, the ventilation channel 32 and the air inlet 33 are in communication through the notch 522 of the second skirt 521 to realize air intake. The diffusion opening formed by the first through hole 512 and the second through hole 523 is in communication with the fragrance spreading opening 34, and the air flow in the fragrance spreading cavity 31 may enter the outside of the fragrance diffusing assembly through the fragrance spreading opening 34 and the diffusion opening, that is, the air path at this time is as follows: the air inlet channel 6- the ventilation channel 32- the notch 522- the air inlet 33-fragrance spreading cavity 31- fragrance spreading opening 34- second through hole 523- first through hole 512- outside of the fragrance diffusing assembly. When the sealing top cover is in the closed position, the ventilation groove and the air inlet 33 are completely staggered. At this time, the air inlet 33 may be sealed by the second skirt 521 of the flexible sealing sleeve 52, and the fragrance spreading opening 34 and the diffusion opening are also completely staggered. The fragrance spreading opening 34 may be sealed by the second skirt 521 of the flexible sealing sleeve 52, and the elastic sealing of the annular wall 35, the fragrance spreading opening 34 and the air inlet 33 may be realized by the sealing top cover 5.

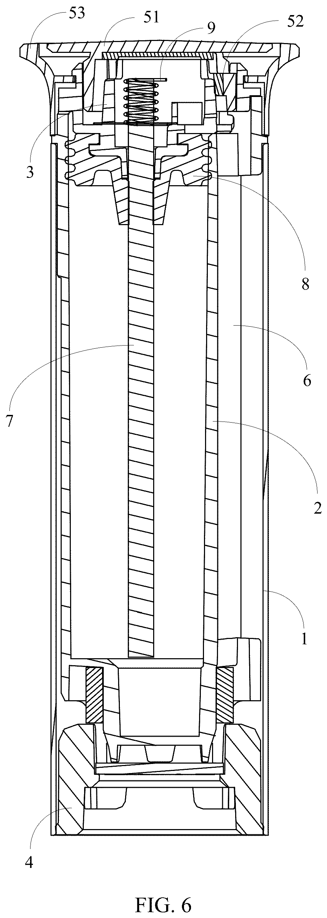

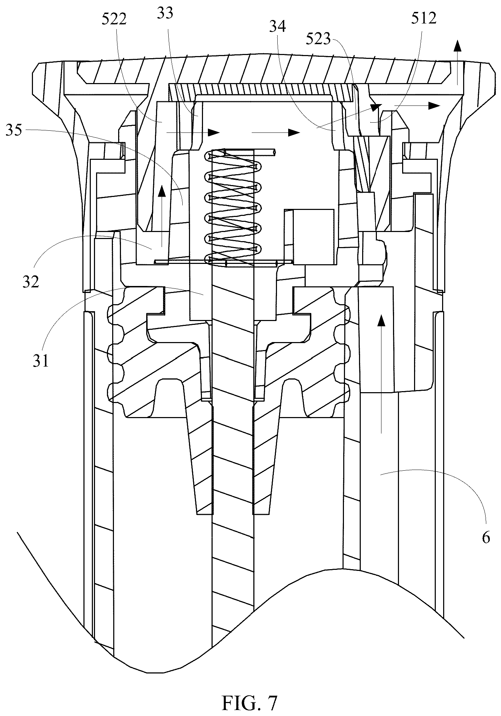

Further, the ventilation channel 32 is in communication with the fragrance spreading opening 34 and the air inlet 33, and the fragrance spreading opening 34 is configured to coincide with the air inlet 33 after rotating 180 degrees around the axial direction of the annular wall 35, that is, the fragrance spreading opening 34 and the air inlet 33 are symmetrically arranged on the annular wall 35. Based on this, it is assumed that the embodiment shown in FIGS. 4 and 5 is a front-installed sealed top cover 5. Then, as shown in FIGS. 6 and 7, when the sealed top cover 5 is rotated 180 degrees on the basis of the embodiment shown in FIGS. 4 and 5, the fragrance spreading opening 34 in the embodiment shown in FIGS. 4 and 5 becomes an air inlet in the embodiment shown in FIGS. 6 and 7, and the air inlet 33 in the embodiment shown in FIGS. 4 and 5 becomes a fragrance spreading opening in the embodiment shown in FIGS. 6 and 7, that is, the air path at this time is: the air inlet 6-the air inlet channel 32- the notch 522- the air inlet 33-the fragrance spreading cavity 31-the fragrance spreading opening 34- the second through hole 523- the first through hole 512- the outside of the fragrance diffusing assembly.

In this way, the wind path designs may be realized under the condition that the sealing top cover 5 is installed forward or backward, so as to realize fragrance spreading and reduce the installation requirements.

As shown in FIGS. 2, 4 and 6, in the above-mentioned embodiments, the fragrance diffusing assembly further includes a hard fiber fragrance spreading stick 7 and a sealing ring 8, which seals the open end of the oil storage cavity 21 and is connected with the mounting bracket 3, and the sealing ring 8 is clamped and fixed with the mounting bracket 3. The hard fiber fragrance spreading stick 7 extends out from the sealing ring 8 and is partially located in the fragrance spreading cavity 31, and the hard fiber fragrance spreading stick 7 and the sealing ring 8 are in interference fit with each other. In this way, the strength may be improved by using the hard fiber fragrance spreading stick 7, which is beneficial to reducing the risk of fracture of the hard fiber fragrance spreading stick 7 in the process of interference assembly between the hard fiber fragrance spreading stick 7 and the sealing ring 8.

Further, the fragrance diffusing assembly also includes a heating wire 9, and the heating wire 9 is wound around one end of the hard fiber fragrance spreading stick 7 located in the fragrance spreading cavity 31, and is fixedly connected with the mounting bracket 3, so that on the one hand, the heating wire 9 may be fixed through the mounting bracket 3, and on the other hand, the fragrant oil may be heated by the heating wire 9 to spread the fragrance.

Based on the technical solution of the present invention, there is also provided a fragrance diffuser, which may include a main unit and the fragrance diffusing assembly described in any of the foregoing embodiments, and the main unit is electrically connected with the fragrance diffusing assembly, so as to realize the fragrance spreading control of the fragrance diffuser through the main unit, such as control of a fragrance spreading duration, a fragrance spreading concentration and the like. A detachable connection mode may be adopted between the main unit and the fragrance diffusing assembly, so as to replace the fragrance diffusing assembly or add the fragrance liquid after the fragrance liquid is used up. When the main unit is detachably connected with the fragrance diffusing assembly, electrical conduction between the main unit and the fragrance diffusing assembly may be realized when the fragrance diffusing assembly is assembled with the main unit. The fragrance diffuser may be a vehicle-installed fragrance diffuser or a household fragrance diffuser, which are not limited by the present invention.

Other embodiments of the present invention will easily occur to those skilled in the art after considering the specification and practicing the present invention disclosed herein. The present invention is intended to cover any variations, uses or adaptations of the present invention, which follow the general principles of the present invention and include common sense or common technical means in this technical field that are not disclosed in the present invention. The specification and examples are to be regarded as illustrative only, with the true scope of the invention being indicated by the following claims.

It should be understood that the present invention is not limited to the precise structure described above and shown in the drawings, and various modifications and changes may be made without departing from its scope. The scope of the present invention is limited only by the appended claims.

## Claims

1. A fragrance diffusing assembly, comprising:
an oil storage member (2) having an oil storage cavity (21) for storing fragrance liquid;
a mounting bracket (3) assembled at an open end of the oil storage cavity (21), and comprising an annular wall (35) enclosing a fragrance spreading cavity (31) in communication with the oil storage cavity (21); and
a sealing top cover (5) rotatably arranged at a top of the annular wall (35) and in elastic contact seal with a top end face and an outer wall surface of the annular wall (35); wherein when the sealing top cover (5) is in an open position, fragrance in the fragrance spreading cavity (31) diffuses to outside of the fragrance diffusing assembly; and when the sealing top cover (5) is in a closed position, the sealing top cover (5) seals the fragrance spreading cavity (31).

2. The fragrance diffusing assembly according to claim 1, wherein a contact surface of the sealing top cover (5) in contact with the top end face of the annular wall (35) and a contact surface of the sealing top cover (5) in contact with the outer wall surface of the annular wall (35) are elastic contact surfaces.

3. The fragrance diffusing assembly according to claim 1, wherein the sealing top cover (5) comprises a hard outer cover (51) and a flexible sealing sleeve (52) fixedly arranged in the hard outer cover (51), and the flexible sealing sleeve (52) is in elastic sealing contact with the top end face and the outer wall surface of the annular wall (35).

4. The fragrance diffusing assembly according to claim 3, wherein the hard outer cover (51) comprises a first skirt (511); and
the flexible sealing sleeve (52) comprises a second skirt (521) fitted with an inner side of the first skirt (511), and the second skirt (521) is in elastic sealing contact with the outer wall surface of the annular wall (35).

5. The fragrance diffusing assembly according to claim 4, wherein the first skirt (511) has a first through hole (512), the second skirt (521) has a notch (522) and a second through hole (523), and the first through hole (512) is in communication with the second through hole (523); and
the notch (522) forms a ventilation groove in communication with the fragrance spreading cavity (31), and the first through hole (512) and the second through hole (523) form a diffusion opening in communication with the fragrance spreading cavity (31).

6. The fragrance diffusing assembly according to claim 3, wherein the sealing top cover (5) further comprises a rotating outer frame (53) rotatably installed on the mounting bracket (3) and located at an outer periphery of the hard outer cover (51), and the rotating outer frame (53) is configured to drive the hard outer cover (51) and the flexible sealing sleeve (52) to rotate under action of an external force.

7. The fragrance diffusing assembly according to claim 1 or 3, wherein the mounting bracket (3) further comprises a ventilation channel (32) arranged outside the fragrance spreading cavity (31), the annular wall (35) is located between the ventilation channel (32) and the fragrance spreading cavity (31), and the annular wall (35) further comprises an air inlet (33) and a fragrance spreading opening (34); and
the sealing top cover (5) comprises a ventilation groove and a diffusion opening, wherein the ventilation groove is in communication with the ventilation channel (32) and the diffusion opening is in communication with the outside of the fragrance diffusing assembly; when the sealing top cover (5) is turned to the open position, the ventilation groove is in communication with the air inlet (33) and the fragrance spreading opening (34) is in communication with the diffusion opening; when the sealing top cover (5) is turned to the closed position, the ventilation groove is completely staggered from the air inlet (33), and the fragrance spreading opening (34) is completely staggered from the diffusion opening, so that the sealing top cover (5) realizes elastic sealing of the fragrance spreading opening (34) and the air inlet (33) of the annular wall (35).

8. The fragrance diffusing assembly according to claim 7, wherein the ventilation channel (32) is in communication with both the fragrance spreading opening (34) and the air inlet (33), and the fragrance spreading opening (34) is configured to coincide with the air inlet (33) after rotating 180 degrees around an axial direction of the annular wall (35).

9. The fragrance diffusing assembly according to any preceding claim, wherein the annular wall (35) comprises an air inlet (33) in communication with the fragrance spreading cavity (31); and the fragrance diffusing assembly further comprises:
a housing (1), wherein the oil storage member (2) is installed in the housing (1) and located at a side of the housing (1), an air inlet channel (6) is formed at another side of the housing (1) and in communication with the air inlet (33) and the outside of the fragrance diffusing assembly, and the air inlet channel (6) is formed by an outer wall of the oil storage member (2) and an inner wall of the housing (1).

10. The fragrance diffusing assembly according to any preceding claim, further comprising a hard fiber fragrance spreading stick (7) and a sealing ring (8), wherein the sealing ring (8) seals the open end of the oil storage cavity (21) and is connected to the mounting bracket (3); the hard fiber fragrance spreading stick (7) extends out from the oil storage member (2) through the sealing ring (8) and is partially located in the fragrance spreading cavity (31); and the hard fiber fragrance spreading stick (7) is in interference fit with the sealing ring (8).

11. A fragrance diffuser, comprising:
the fragrance diffusing assembly according to any one of claims 1 to 10; and
a main unit electrically connected with the fragrance diffusing assembly.
